# EUROPEAN PATENT APPLICATION

(11) **EP 3 366 758 A1**
(43) Date of publication of application: **29.08.2018**
(21) Application number: 16857461.4
(22) Date of filing: 19.10.2016
(51) Int. Cl.: C12M 1/00

(54) **CELL TREATMENT DEVICE**

(30) Priority: 20.10.2015 JP 2015206193
(71) Applicant: Rohto Pharmaceutical Co., Ltd., Osaka-shi Osaka 544-8666 (JP)
(72) Inventor: KOIKE, Tetsuo, Osaka-shi Osaka 544-8666 (JP); TAKIMOTO, Masahiro, Osaka-shi Osaka 544-8666 (JP); YAGI, Yoshiki, Kuse-gun Kyoto 613-0036 (JP)
(74) Representative: DTS Zürich
(86) International application number: PCT/JP2016/080943
(87) International publication number: WO 2017/069148

(57) **Abstract**

Provided is a cell treatment apparatus that includes: an isolator 3 that has an inner space maintained in aseptic conditions and is configured to treat cells in the inner space; trays 29 and 30 that are each configured to house plural kinds of articles, which include reagent containers with articles and reagents therein for use in treatment of cells in the inner space of the isolator 3, while positioning them; and pass boxes 4 that are configured to respectively carry the trays 29 and 30 with the plural kinds of articles housed therein into the isolator 3.

## Description

### CROSS-REFERENCE TO RELATED APPLICATION

This application claims priority to Japanese Patent Application No. 2015-206193, the disclosure of which is incorporated herein by reference in its entirety.

### FIELD

The present invention relates to a cell treatment apparatus for cell treatment.

### BACKGROUND

In recent years, cell culture is performed using tissues and cells of various sites of human body, fertilized eggs, or the like, and the cultured cells have been put to practical use for regenerative medicine. In the cell culture, it is important to prevent contamination of cells by bacteria or the like during the culture. Therefore, a cell treatment apparatus that enables the culture of cells in an environment that can maintain thereinside in aseptic conditions has been already proposed.

The aforementioned cell treatment apparatus includes an equipment installing section with an operating robot arranged therein, and a conveying unit for conveying containers (articles) for use in treatment cells to the equipment installing section side. This conveying unit is constituted by a glovebox for temporal placement of the containers, and a communication section for connecting the glovebox with the equipment installing section. Arranged inside the conveying unit are a carriage, on which containers put in the glovebox are mounted, and rails having a length extending between the glovebox and the communication section (for example, Patent Literature 1)

### CITATION LIST

### Patent Literature

Patent Literature 1: JP 2008-239168 A (see Fig. 3 and Fig. 4)

### SUMMARY

### Technical Problem

Meanwhile, large kinds of containers are needed for containers such as reagent containers in which articles and reagents for use in cell treatment are contained. In this regard, Patent Literature 1 mentioned above is configured so that the same kind of containers are housed in a dedicated stand, and the stand with the plurality of containers housed therein is placed on the carriage to be conveyed toward the equipment installing section. Therefore, different kinds of containers cannot together be conveyed, which causes increase in the number of times that containers are conveyed by the carriage as the kinds of containers are increased. As a result, it take a long time to convey the containers, which poses a disadvantage of deteriorating the working efficiency.

In view of the above circumstances, it is an object of the present invention to provide a cell treatment apparatus that can shorten the time for conveying the articles.

### Solution to Problem

A cell treatment apparatus according to the present invention includes: an isolator that has an inner space maintained in aseptic conditions and is configured to treat cells in the inner space; trays that are each configured to house plural kinds of articles for use in treatment of cells in the inner space of the isolator, while positioning them; and a pass box that is configured to carry the trays with the plural kinds of articles housed therein into the inner space of the isolator.

The cell treatment apparatus according to the present invention may be configured so that the isolator includes in the inner space a guide unit that guides the movement of the trays carried into the inner space of the isolator in a direction crossing the carrying-in direction.

The cell treatment apparatus according to the present invention may be configured so that the isolator includes in the inner space a robot for handling the articles housed inside of each of the trays carried into the isolator from the pass box, and the robot is configured to move the trays carried into the inner space in a direction crossing the carrying-in direction.

The cell treatment apparatus according to the present invention may be configured so that a plurality of pass boxes are provided.

### BRIEF DESCRIPTION OF DRAWINGS

Fig. 1 is a front view of an apparatus to produce cultured cell products of the present invention.
Fig. 2 is a plan view of the aforementioned production apparatus.
Fig. 3 is a view of the aforementioned production apparatus as seen from the outlet side.
Fig. 4 is an explanatory diagram showing the state immediately before a vessel cap is opened by a robot arm.
Fig. 5 is a view as seen from the direction of arrow A-A in Fig. 2.

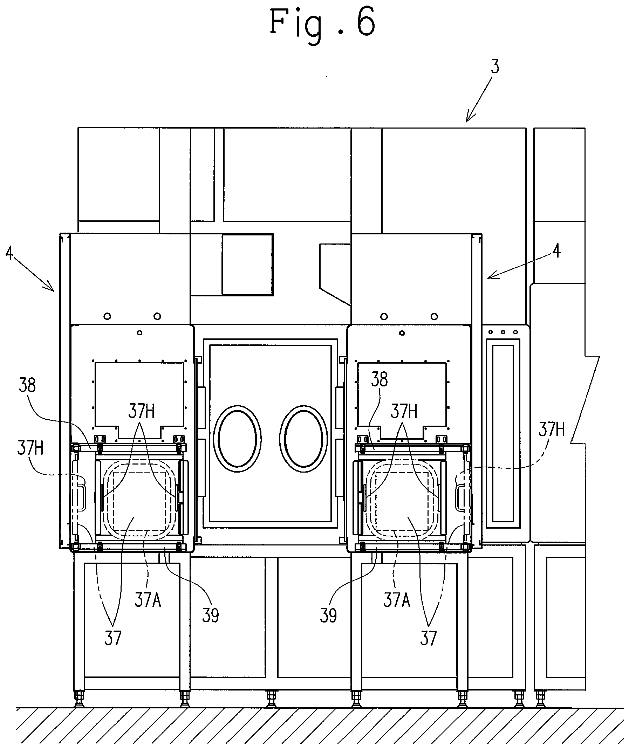

Fig. 6 is a cross sectional view taken along line B-B in Fig. 2.
Fig. 7 is a cross sectional view taken along line C-C in Fig. 2.

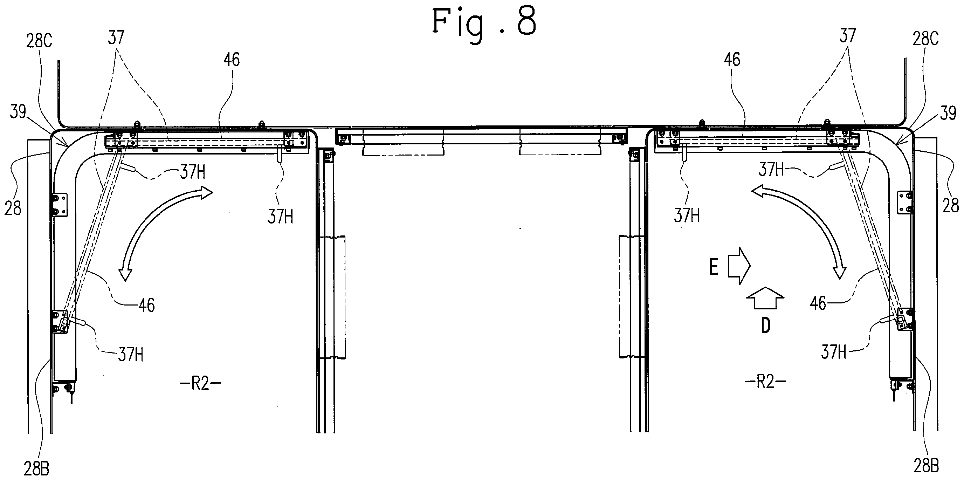

Fig. 8 is a plan view showing a cover slide structure included in a decontamination chamber of a pass box.
Fig. 9 is a view as seen from the direction of arrow D-D in Fig. 8.
Fig. 10 is a view as seen from the direction of arrow E-E in Fig. 8.
Fig. 11a is a plan view of a tray that houses containers.
Fig. 11b is a plan view of a tray that houses containers.
Fig. 12a is a plan view showing a configuration of a first carrying-in unit that is configured to carry a first tray from a pass box on a left side into the isolator.
Fig. 12b is a side view of the same.
Fig. 13a is a plan view showing a configuration of a second carrying-in unit that is configured to carry a second tray from a pass box on a right side into the isolator.
Fig. 13b is a side view of the same.
Fig. 14a is a plan view showing a site where trays carried into the isolator are aligned.
Fig. 14b is a front view of the same.
Fig. 15 is a plan view showing the state where the trays carried into the isolator from the pass box on the left side are aligned.

### DESCRIPTION OF EMBODIMENTS

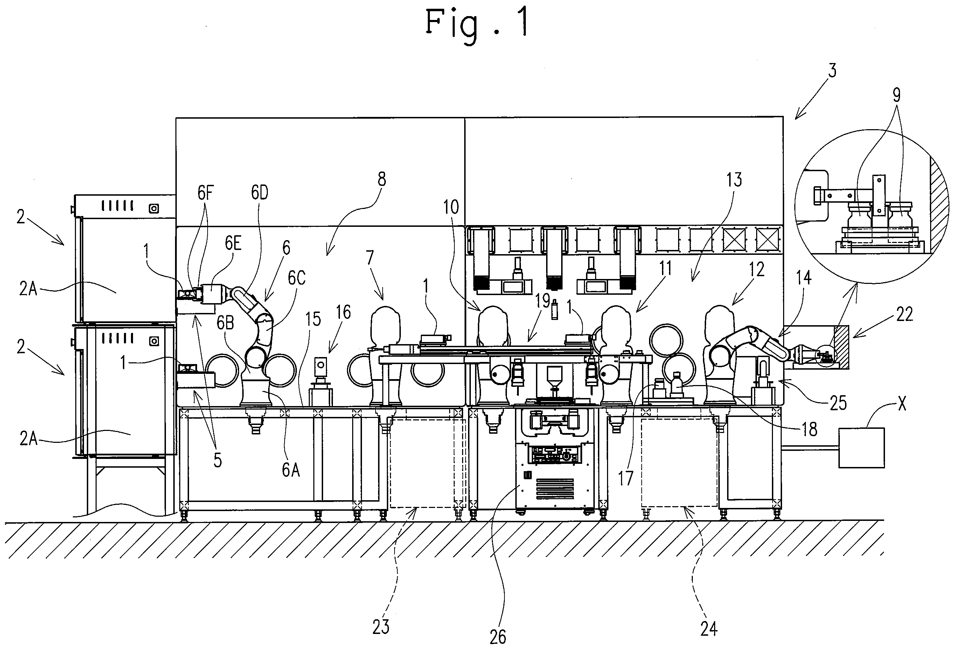

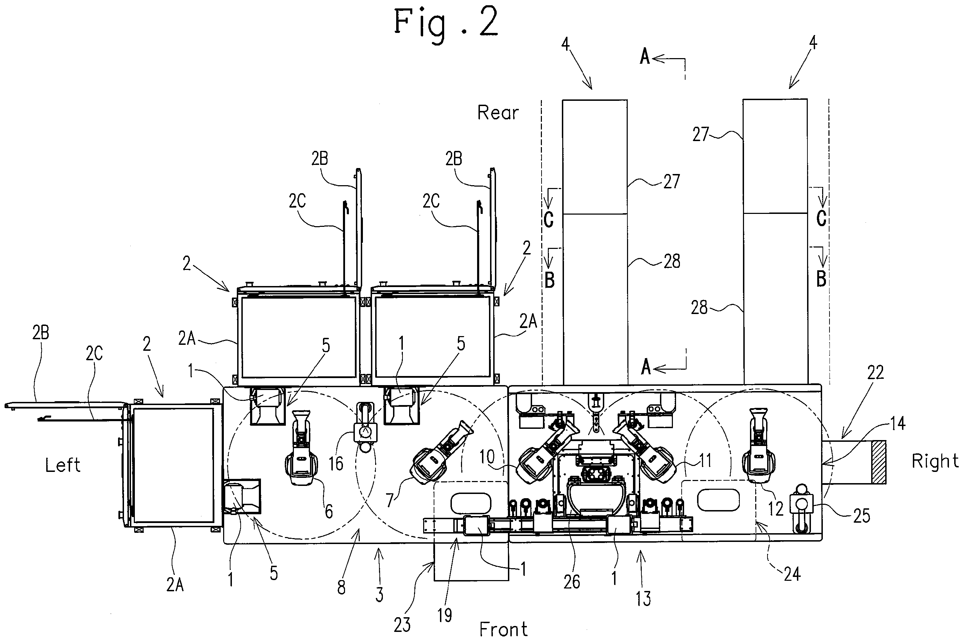

Hereinafter, an apparatus to produce cultured cell products (hereinafter, referred to as production apparatus) that is an example of a cell treatment apparatus of the present invention will be described. In the following description on the front, rear, left, and right directions, the left and right directions correspond to the state shown in Fig. 1 and Fig. 2, and the front and rear directions correspond to the state of Fig. 2 where the lower side corresponds to the "front" and the upper side corresponds to the "rear" (the directions are shown also in Fig. 2).

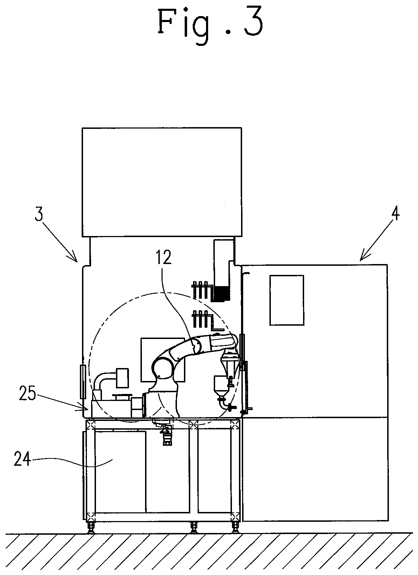

Fig. 1 to Fig. 3 show the production apparatus of this embodiment. The production apparatus includes a plurality of incubators 2, a horizontally elongated isolator 3, and a plurality (two in Fig. 2) of pass boxes 4. An incubator 2 houses a cell culture vessel (hereinafter referred to a culture vessel) 1 that is an article. The isolator 3 is capable of having its inside maintained in aseptic conditions and treats the culture vessel 1 transferred from the incubator 2. The pass boxes 4 are configured to be capable of carrying a reagent container into the isolator 3, in which the reagent container contains an article and a reagent necessary for subdividing and putting-in of cells cultured in the culture vessel 1. As the culture vessel 1, a HYPERFlask (manufactured by Corning Incorporated) capable of culturing cells in multilayers, for example, can be used. Each part is controlled by a control device X schematically shown in Fig. 1. The control device X may be provided integrally with the production apparatus or may be a separate body (such as a personal computer) connected to the production apparatus in a wired or wireless manner. Further, it is also possible to provide the control device X integrally with the production apparatus and provide only an operation section of the control device X that is operated by an operator (such as a tablet terminal) as a separate body from the production apparatus.

The incubators 2 are provided while being vertically stacked in two stages as shown in Fig. 1. Six units of the incubators 2 are provided in total, at three points in total including one point at the left end of the isolator 3 and two points in the left end part behind the isolator 3, as shown in Fig. 2. Two incubators 2, 2 in the upper stage and the lower stage have the same configuration. Each of the incubators 2 is provided with racks (not shown) capable of housing a large number of the culture vessels 1 within a casing 2A. The casing 2A has a box shape with one lateral side opening so that the culture vessels 1 can be taken in and out through the lateral side. Two doors 2B and 2C configured to close the opening on the one lateral side of the casing 2A are attached to the casing 2A so as to be freely openable. The inside door 2C is formed with a transparent material, so that the number of the culture vessels 1 housed therein and the state of the cell culture can be checked only by opening the outside door 2B while the opening is closed by the inside door 2C. Further, carbon dioxide gas for adjusting the culture atmosphere is configured to be supplied into the incubators 2. Further, the culture vessels 1 housed on the racks inside the incubators 2 are configured to be delivered onto a plurality of mounting tables 5 provided in the isolator 3 by a delivery mechanism, which is not shown. Six units of the mounting tables 5, which is the same number as the number of the incubators 2, are arranged corresponding to the incubators 2.

As described above, the isolator 3 is horizontally elongated (in this embodiment, it is rectangular in planer view), where one set (two units on the upper and lower sides) of incubators 2 is located on the short side of the isolator 3 (in this embodiment, the left side), and a plurality of sets (in this embodiment, two sets of incubators 2) are located on the longitudinal side (in this embodiment, the rear side). This configuration can reduce the size of the production apparatus without decreasing the number of cultured cells.

The isolator 3 includes an observation section 8, a processing section 13, and an outlet 14. The observation section 8 includes two first robot arms 6 and 7 configured to move the culture vessels 1 to an observation position so that the degree of growth in the culture vessels 1 taken out of the incubators 2 is checked. The processing section 13 is provided continuously with the observation section 8. The processing section 13 includes three second robot arms 10, 11, and 12. The second robot arms 10, 11, and 12 are configured to transfer cells in the culture vessels 1 that have a specified number of cells out of the culture vessels 1 observed in the observation section 8 into a large number of product containers 9 (such as vial containers, see the enlarged view of Fig. 1), which have been carried in from the pass boxes 4. The outlet 14 is configured to allow the large number of product containers 9 into which the cells have been transferred to be taken out therethrough. A large number of work gloves (not shown) that allow the operator to perform operations by putting their hands into the isolator 3 are attached onto the front and rear walls of the isolator 3. As shown in Fig. 2, the five robot arms 6, 7, 10, 11, and 12 are aligned in a straight line extending in the left and right directions along the longitudinal direction of the isolator 3.

With reference to the left and right directions, the first robot arm 6 on the left side corresponds to one set of incubators 2 located on the short side of the isolator 3 (in this embodiment, on the left side) and one set of incubators 2 on the left side out of the sets of incubators 2 located on the longitudinal side (in this embodiment, on the rear side). The first robot arm 6 on the left side can handle the culture vessels 1 that are housed in these incubators 2 (the range that can be reached by each robot arm (in planer view) is shown in Fig. 2 with a dashed-double-dotted circle). Further, the first robot arm 7 on the right side corresponds to one set of incubators 2 on the right side out of the sets of incubators 2 located on the longitudinal side of the isolator 3. The first robot arm 7 on the right side can handle the culture vessels 1 housed in the incubators 2.

With reference to the left and right directions, the second robot arm 10 on the left side and the second robot arm 11 in the middle correspond to the pass box 4 on the left side out of the pass boxes 4 located on the longitudinal side of the isolator 3 (in this embodiment, on the rear side). The second robot arm 10 on the left side and the second robot arm 11 in the middle can handle reagent containers in which articles and reagents are contained, the reagent containers being to be housed (or having been housed) in the pass box 4.

The second robot arm 12 on the right side corresponds to the pass box 4 on the right side out of the pass boxes 4 located on the longitudinal side of the isolator 3 (in this embodiment, on the rear side) and a box 22 for carrying out the product containers 9. The second robot arm 12 on the right side can handle reagent containers in which articles and reagents are contained, the reagent containers being to be housed (or having been housed) in the pass box 4, and the product containers 9 to be housed in the box 22.

As seen from the overlapping of the dashed-double-dotted circles shown in Fig. 2, the five robot arms 6, 7, 10, 11, and 12 are arranged in a positional relationship so as to be capable of passing articles to each other.

In this way, the robot arms 6, 7, 10, 11, and 12 are located within the isolator 3, thereby enabling each of the robot arms 6, 7, 10, 11, and 12 to act on the incubators 2, the isolator 3, the pass boxes 4, and the box 22 according to the purpose. Thus, according to the production apparatus of this embodiment, it is possible to improve the working efficiency and contribute to mass production of cultured cell products.

The first robot arms 6 and 7 and the second robot arms 10, 11, and 12 in this embodiment have the same configuration. Therefore, the description for the first robot arm 6 located at the left end will be applied to the description for each of the first robot arm 7, and the second robot arms 10, 11, and 12. The first robot arm 6 is constituted by articulated robot arm. The first robot arm 6 includes a fixed part 6A fixed to a base member 15 of the isolator 3, a base part 6B that is pivotable about the vertical axis at the distal end part of the fixed part 6A, a first arm 6C that is swingable about the horizontal axis at the distal end part of the base part 6B, a second arm 6D that is swingable about the horizontal axis at the distal end part of the first arm 6C, a third arm 6E that is swingable about the horizontal axis at the distal end part of the second arm 6D, and a pair of grips 6F, 6F that are attached to the distal end of the third arm 6E so as to be opposed thereto. The pair of grips 6F, 6F are configured to be capable of moving close to and away from each other. The articulated first robot arms 6 and 7 can hold the culture vessels 1 delivered from the incubators 2 using the pair of grips 6F (see Fig. 1) and move them to a microscope 16 at the observation position. The second robot arms 10, 11, and 12 are configured to hold such as a centrifuge tube 17 and a preparation tank 18 shown in Fig. 1 in addition to the culture vessels 1 so as to be capable of performing various processes.

The microscope 16 located at an observation position is arranged between the two first robot arms 6 and 7. According to such an arrangement, it is possible to move the culture vessels 1 to the microscope 16 using the first robot arm 6 on the left side so as to observe the cells, and as a result of the observation, it is possible to hold the culture vessels 1 that have been determined to have a specified number of cells so as to rapidly move them to the processing section 13 side, using the first robot arm 7 on the right side. That is, the first robot arm 6 on the left side mainly performs the operation to move the culture vessels 1 to the microscope 16, and the first robot arm 7 on the right side performs the operation to move the culture vessels 1 that have been determined to have a specified number of cells toward the processing section 13 side. These operations by the first robot arms 6 and 7 can accelerate the operation speed. The determination on whether the culture vessels 1 have a specified number of cells may be made by counting the number of cells by visual inspection of the operator (human) of the production apparatus or may be made automatically by the control device based on the number of cells calculated by analyzing an image captured by a camera so as to automatically calculate the number of cells. The culture vessels 1 that are delivered from the incubator 2 located opposed to the first robot arm 7 on the right side are held by the first robot arm 7 on the right side to be moved to the microscope 16. Further, a microscope 25 is provided also in the processing section 13. The object observed by the microscope 25 is held by the second robot arm 12 on the right end to be moved.

The culture vessels 1 after the observation are conveyed not only by being directly passed from the first robot arm 7 on the right side to the second robot arms 10 arranged at the left end of the processing section 13. For example, in the case where the second robot arm 10 is in an operation, the culture vessels 1 are conveyed by a conveying apparatus 19 to a position where the second robot arm 10 at the left end of the processing section 13 or the second robot arm 11 arranged at horizontal center of the processing section 13 can grip them. The conveying apparatus 19 is provided along the front sidewall of the isolator 3 and is set to a length that allows the conveying apparatus 19 to convey them from the right end part of the observation section 8 of the isolator 3 to the horizontal center of the processing section 13. Accordingly, when the first robot arm 7 on the right side passes the culture vessels 1 after the observation to the conveyance starting end part of the conveying apparatus 19, the conveying apparatus 19 conveys the culture vessels 1 to the position where one of the two second robot arms 10 and 11 can grip them.

The conveying apparatus 19 is provided corresponding to at least one robot arm (in this embodiment, the first robot arm 7) located in the observation section 8 and a plurality of robot arms (in this embodiment, the two second robot arms 10 and 11) located in the processing section 13. The first robot arm 7 can directly deliver the articles to the second robot arm 10. The conveying apparatus 19 can deliver the articles to the first robot arm 7 and the third robot arm 11 between which direct delivery of the articles is impossible. Therefore, even in the case where the articles cannot be delivered from the first robot arm 7 to the third robot arm 11 via the second robot arm 10 due to the second robot arm 10 being in operation, the articles can be delivered from the first robot arm 7 to the third robot arm 11 via the conveying apparatus 19. Therefore, the articles can be conveyed in parallel (via a plurality of routes) within the isolator 3. Accordingly, the working efficiency within the isolator 3 can be improved, and thus the productivity can be improved.

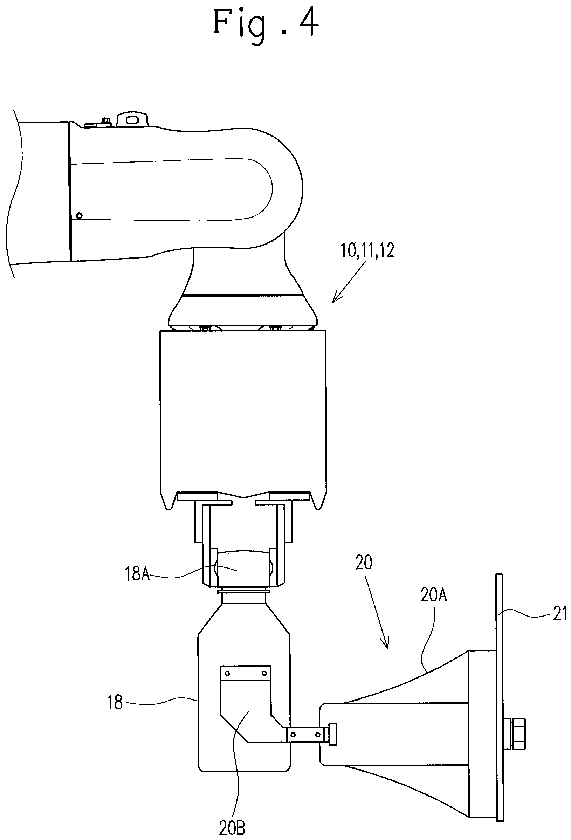

In the processing section 13, three units of the second robot arms 10, 11, and 12 are arranged at equal intervals, and the intervals are set to be smaller than the interval between the two first robot arms 6 and 7, so that the speed of various processes performed between the second robot arms 10 and 11 or 11 and 12 is higher. As shown in Fig. 4, an immovable fixed auxiliary arm 20 is provided at a position in the vicinity of each of the second robot arms 10, 11, and 12 and below each of the second robot arms 10, 11, and 12. The auxiliary arm 20 includes a fixed part 20A fixed to a fixing member 21, and a pair of grips 20B, 20B (in Fig. 4, only the grip 20B on the front side is shown) attached so as to be capable of moving close to and away from the fixed part 20A. Fig. 4 shows the state where, for example, after the upper end part of the preparation tank 18 is held by the second robot arm 10, 11, or 12, so as to be moved to a position where it can be gripped by the pair of grips 20B, 20B of the auxiliary arm 20, the lower end part of the preparation tank 18 is gripped by the pair of grips 20B of the auxiliary arm 20. In this way, a cap 18A of the preparation tank 18 can be opened or closed by the single second robot arm 10, 11, or 12. Further, a program to open and close screw caps that are provided on a plurality of types of containers is stored in the second robot arms 10, 11, and 12, so that the second robot arms 10, 11, and 12 can open and close the screw caps provided on the plurality of types of containers. Therefore, it is not necessary to unify the types of containers into the same type, and thus the production apparatus of cultured cell products can be easily achieved. Also in the first robot arms 6 and 7, such a program may be stored.

The two pass boxes 4, 4 are provided to be continuous with the rear wall of the processing section 13. One (on the left side) of the pass boxes 4 is arranged so that the articles can be carried therein passing through between the second robot arm 10 located at the left end and the second robot arm 11 located at the center. Examples of the articles include a plurality of types of containers including the product containers 9, the culture vessels 1, and the centrifuge tube 17, and the preparation tank 18 that is a container in which drugs are put. The other (on the right side) of the pass boxes 4 is arranged so that the articles are carried to the second robot arm 12 located at the right end. The articles (various kinds of containers) carried from the pass box 4 on the left side are handled by the second robot arm 10 located at the left end and the second robot arm 11 located at the horizontal center, while the articles (containers) carried from the pass box 4 on the right side are handled by the second robot arm 12 located at the right end.

As described above, the isolator 3 is horizontally elongated, in which the plurality (in this embodiment, two) of pass boxes 4 are located on the longitudinal side of the isolator 3 (in this embodiment, on the rear side). This configuration can reduce the size of the production apparatus without limiting the amount of articles to be carried into the isolator 3.

The opening of the outlet 14 is configured to have a size such that the second robot arm 12 located at the right end can easily enter therethrough. The outlet 14 is provided with a freely openable electric shutter (not shown) and is provided continuously with the box 22 that forms a space in which the product containers 9 moved through the outlet 14 to the outside of the isolator 3 are kept for a while.

The processing section 13 includes a first transfer processing unit, a separation processing unit, and a second transfer unit. The first transfer processing unit is configured to transfer a cell-containing liquid housed in the culture vessels 1 received from the first robot arm 7 into the centrifuge tube 17 using the second robot arm 10. The separation processing unit is configured to separate the cells and a liquid portion by subjecting the centrifuge tube 17 to a centrifuge 26 using the second robot arm 10. The second transfer unit is configured to transfer a specified number of cells within the centrifuge tube 17 into a large number of the product containers 9 while a preservative solution (cryopreservation solution) is put into the centrifuge tube 17 after removing at least part of the liquid portion separated in the separation processing unit from the centrifuge tube 17, using the second robot arm 10. In the description of this embodiment, the term "cell-containing liquid" simply means a "liquid containing cells" and is not limited to a liquid in a specific state.

The processing section 13 includes a medium-replacing unit configured to replace the culture medium within the culture vessels 1 taken out of the incubators 2 using the first robot arm 7. The medium-replacing unit is configured to open the caps of the culture vessels 1 received by the second robot arm 10 from the first robot arm 7, to dispose of the culture medium within the culture vessels 1, to supply another culture medium into the cell culture vessels 1, to put the caps thereon, and to return them to the first robot arm 7.

The processing section 13 configured as above is capable of performing a first process of thawing frozen cells and seeding them, a second process (passage process) of collecting the cells and seeding them on a large number of culture vessels, and a third process of collecting the cultured cells in the culture vessels after the passage process, subdividing the collected cells, transferring them into the product containers 9, and carrying them out through the outlet 14.

Provided at a position close to the processing section 13 within the observation section 8 is a first disposal part 23 for disposal of waste products, which can be lid-closed, such as the centrifuge tube 17 and the preparation tank 18 (mainly those having a large size), in addition to the cell culture vessels 1, which become unnecessary during the aforementioned processes. Provided at a position close to the box 22 within the processing section 13 is a second disposal part 24 for disposal of waste products (mainly those having a small size, but even for tips, there are large tips such as disposable tips), which cannot be lid-closed and therefore may cause dripping, such as pipette tips (suction openings mounted to pipettes, not shown).

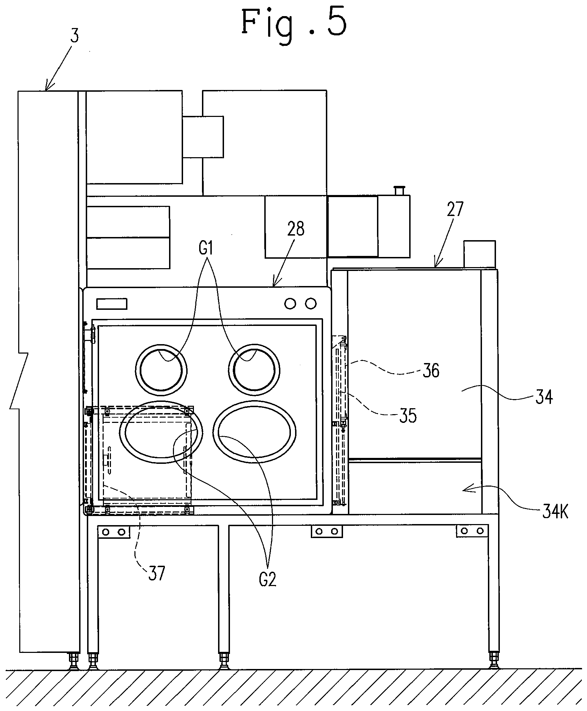

The two pass boxes 4 have the same configuration. Each of the pass boxes 4 includes a first box 27 that constitutes a clean bench chamber for carrying containers from outside thereinto, and a second box 28 that constitutes a decontamination chamber for carrying containers from the clean bench chamber into the isolator 3. As shown in Fig. 5, each of inner side surfaces respectively opposed to the second boxes 28, 28 (only one of them are shown in Fig. 5) has a large number of (four in Fig. 5) glove ports G1 and G2 to enable operation by putting the hand in the decontamination chamber R2.

As shown in Fig. 5, shutters 34 of sliding type (only one of them is shown in Fig. 5) that can slide in the vertical direction are each provided on one lateral side of each of the two opposed clean bench chambers. Each of the shutters 34 is configured to change the opening degree of an opening 34K formed on a lower side. Herein, the opening 34K is set to be substantially one fourth of an area when the shutter 34 is in the fully opened position. Containers are manually passed through the opening 34K from the outside of the clean bench chamber R1 into the clean bench chamber, where containers are subjected to decontamination with alcohol or other treatments.

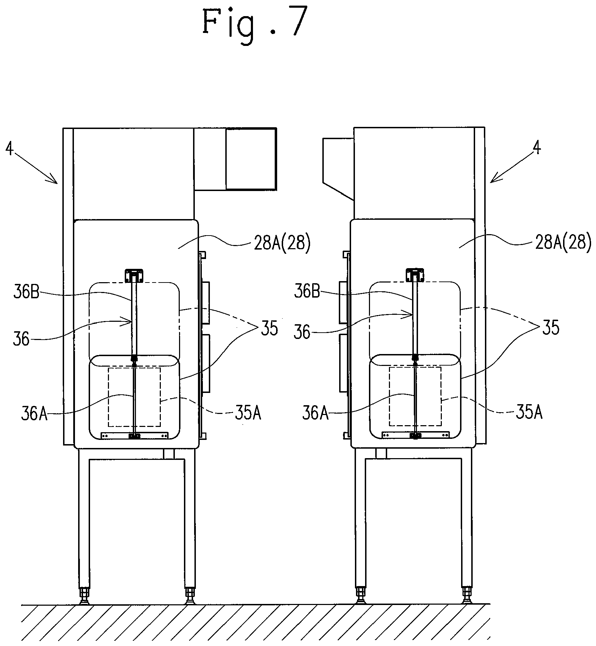

The clean bench chamber includes a decontamination chamber opening 35A that is openable and closable for carrying containers into the decontamination chamber. As shown in Fig. 7, this decontamination chamber opening 35A can be opened and closed by moving up and down a first cover 35 using an air cylinder 36. The first cover 35 is formed by a plate-shaped member having a substantially square shape, and is vertically movably supported to a rear wall 28A of the second box 28 by a guide mechanism (not shown). The air cylinder 36 has a cylinder tube 36B that is fixed to the rear wall 28A of the second box 28 with a proximal end of the cylinder tube 36B upwardly oriented, and has a piston rod 36A that is connected to a lower end of the first cover 35 with a distal end of the piston rod 36A oriented downwardly. The piston rod 36A of the air cylinder 36 is retracted to slide the first cover 35 upwardly so that the decontamination chamber opening 35A is brought into an opened state, and the piston rod 36A of the air cylinder 36 in the retracted state is extended to slide the first cover 35 downwardly, which has been slid upwardly, so that the decontamination chamber opening 35A is brought into a closed state.

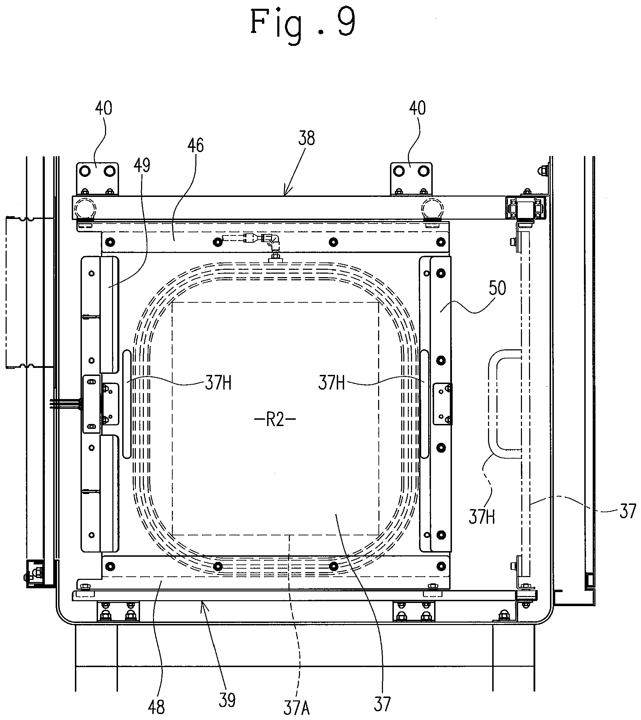

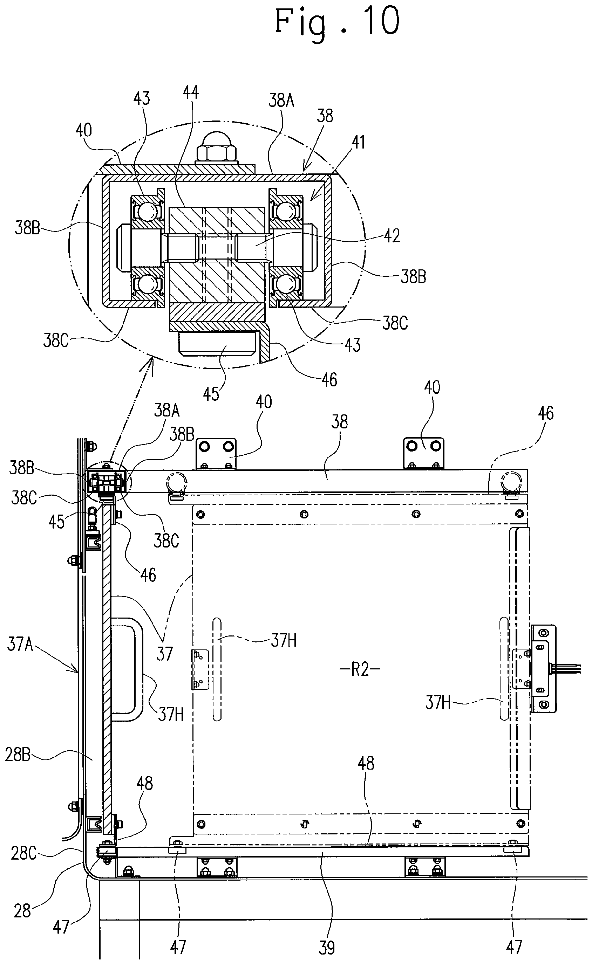

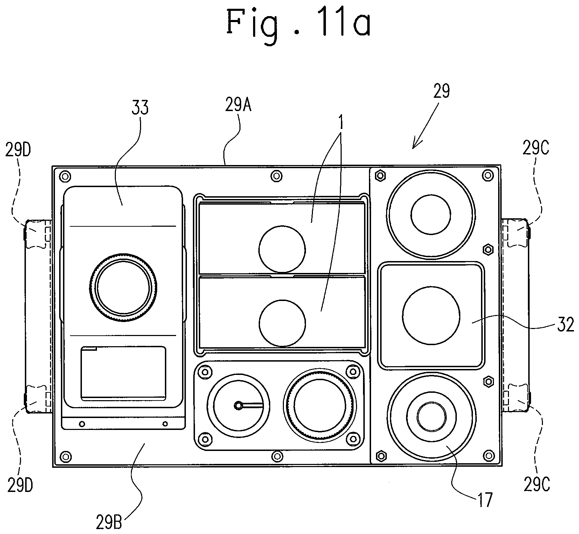

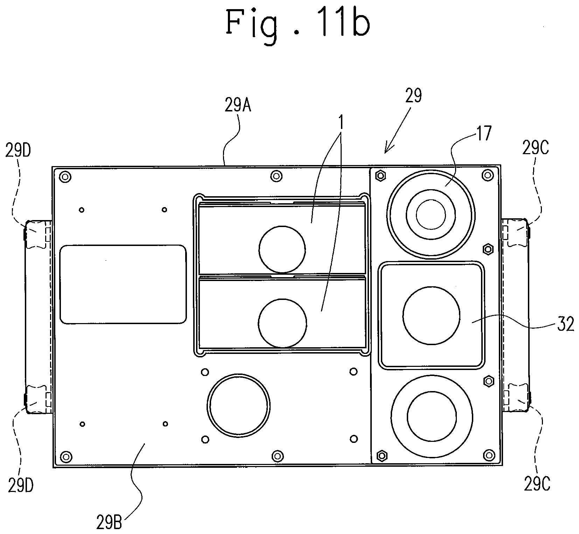

The decontamination chamber includes an isolator opening 37A that can be opened and closed, through which containers carried from the clean bench chamber into the decontamination chamber is carried into the isolator 3. As shown in Fig. 9 and Fig. 10, the isolator opening 37A can be opened and closed by sliding a second cover 37 in the lateral direction. The second cover 37 is formed by a plate-shaped member having a substantially square shape, and includes handles 37H, 37H for opening and closing operation at both ends in the slide direction. The second cover 37 is configured so as to be able to move in the lateral direction while having its upper and lower ends supported by a pair of upper and lower guide rails 38 and 39. The upper guide rail 38 and the lower guide rail 39 are arranged to oppose to each other in the vertical direction, and are formed respectively by L-shaped members fixed over a front wall 28B and a left side wall 28C that are two adjacent side walls of the second box 28 constituting the decontamination chamber. The upper guide rail 38 includes a top plate part 38A fixed to four (only three are shown in Figs. 11) brackets 40 fixed to the two side walls 28B and 28C, a pair of vertical plate parts 38B, 38B extending downward from both ends in the width direction of the top plate part 38A, and a pair of horizontal plate parts 38C, 38C that respectively extend from lower ends of the pair of vertical plate parts 38B, 38B to be close to each other. A pair of moving bodies 41, 41 are movably provided inside the upper guide rail 38, and an upper end of the second cover 37 is connected to the pair of moving bodies 41, 41. Each of the moving bodies 41 includes a pair of bearings 43, 43 that are mounted on the pair of horizontal plate parts 38C, 38C and coupled to each other through lateral shafts 42, a spacing member 44 for holding a space between the bearings 43, 43 and having the lateral shafts 42 extending therethrough, and a pin 45 that is attached to the spacing member 44 while extending therethrough movably around a vertical axis. In each of the moving bodies 41, an upper frame body 46 having a reversed L-shape, which is fixed to an upper end of the second cover 37 with screws, is fastened and fixed to the spacing member 44 by the pin 45. Accordingly, the second cover 37 can smoothly change its moving direction without deflecting during moving through a curved portion of the upper guide rail 38 since the second cover 37 can smoothly move along the upper guide rail 38 by the rotation of the pair of bearings 43, while rotating around the axis of the pin 45. The lower guide rail 39 has a substantially U-shape with its upper end opening, and a pair of left and right rollers 47, 47, which are rotatable around the vertical axis, are provided within the lower guide rail 39. The pair of left and right rollers 47, 47 are coupled, rotatably around the vertical axis, to a lower frame body 48 that has a L-shape and is fixed to a lower end of the second cover 37 with screws. Accordingly, the second cover 37 can smoothly change its moving direction without deflecting during moving through a curved portion of the lower guide rail 39 since the second cover 37 can smoothly move along the lower guide rail 39 by the rotation of the pair of left and right rollers 47, 47.

The reference numeral 49 shown in Fig. 10 represents a stopper member, to which the second cover 37 abuts when it is located at the closing position, and the reference numeral 50 represents a stopper member, to which the second cover 37 abuts when it is located at the opening position.

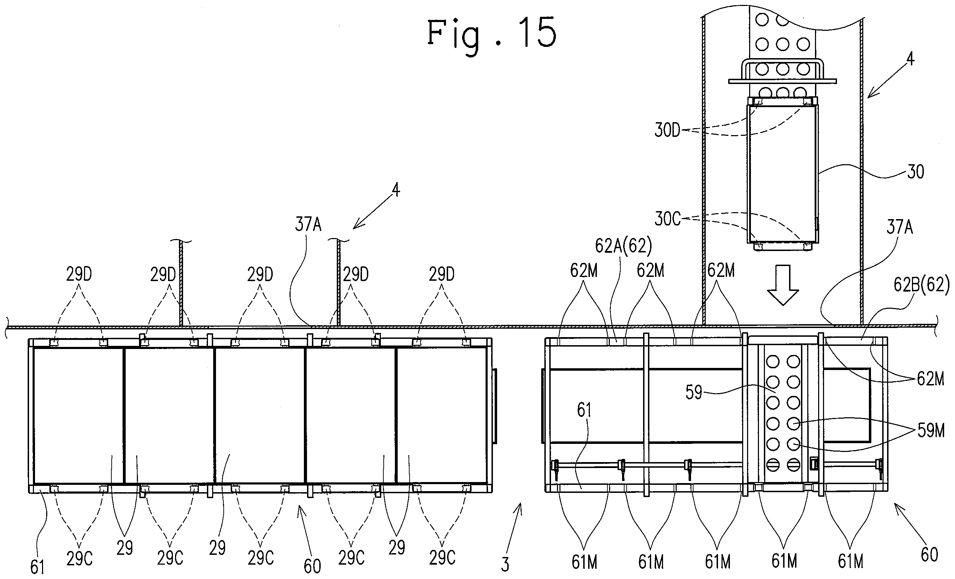

The culture vessel 1, the centrifuge tube 17, the preparation tank 18 and the like, which are various kinds of containers for use in the isolator 3, are placed in the pass box 4 on the left side, then housed in a first tray 29 shown in Fig. 11a and Fig. 11b, and then the first tray 29 is carried into the isolator 3 through a carrying-in device 31. The first tray 29 is constituted by a housing body 29A of a box type having a rectangular shape in planer view with its upper side opening, a partitioning plate 29B that is configured to house various kinds of containers while positioning them within the housing body 29A, and pairs of front and rear rotating rollers 29C, 29C and 29D, 29D that are mounted at both ends in the front-rear direction (carrying-in direction) of the housing body 29A with a certain interval in the width direction. Specifically, Fig. 11a shows the case where one centrifuge tube 17, one waste liquid tank 32, two culture vessels 1, 1, and one PBS (phosphate buffered saline) solution tank 33 are housed in the first tray 29. Holes matching in shape with these various kinds of containers (holes capable of receiving various kinds of containers and positioning the same) are formed in the partitioning plate 29B. In Fig. 11b, one centrifuge tube 17, one waste liquid tank 32, and two culture vessels 1, 1 are housed in the first tray 29. Holes matching in shape with these various kinds of containers (holes capable of receiving various kinds of containers and positioning the same) are formed in the partitioning plate 29B. The first tray 29 of Fig. 11a and the first tray 29 of Fig. 11b have the same configuration for the housing body 29A and the pairs of the front and rear rotating rollers 29C, 29C and 29D, 29D, while having a slight difference in those holes formed in the partitioning plate 29B. The first trays 29 are carried into the isolator 3 from the pass box 4 on the left side. A second tray 30 to be carried from the pass box 4 on the right side is smaller in width than the first tray 29 to be carried from the pass box 4 on the left side into the isolator 3 (Fig. 15 shows the second tray 30), and houses containers, which mainly have a smaller size than the containers housed in the first tray 29. The second tray 30 houses, for example, a cell counter, a microplate, a pipette tip, and the like.

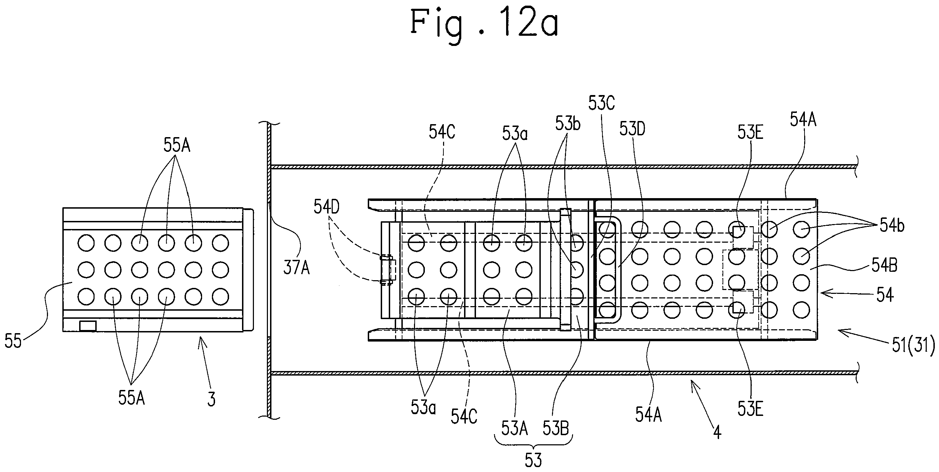

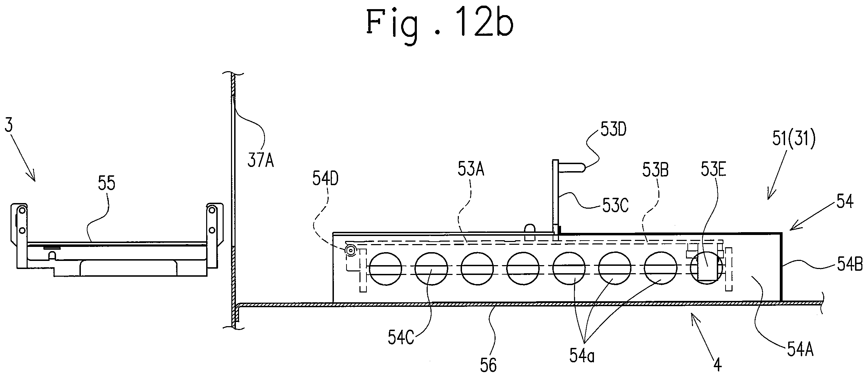

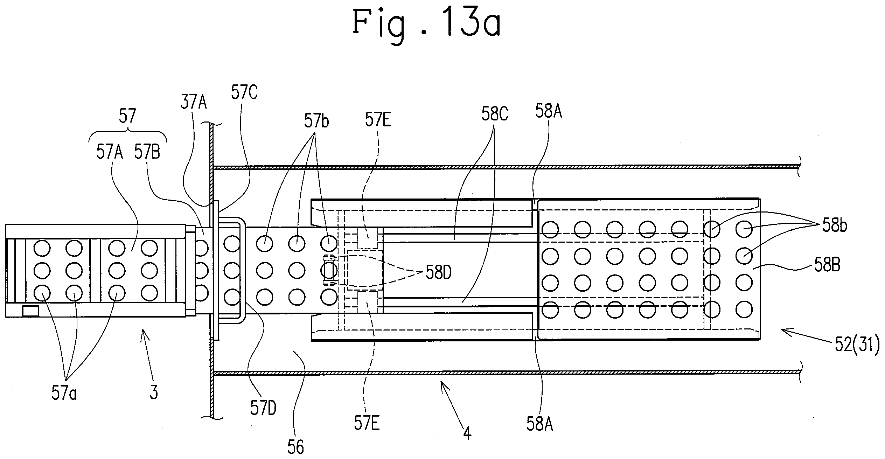

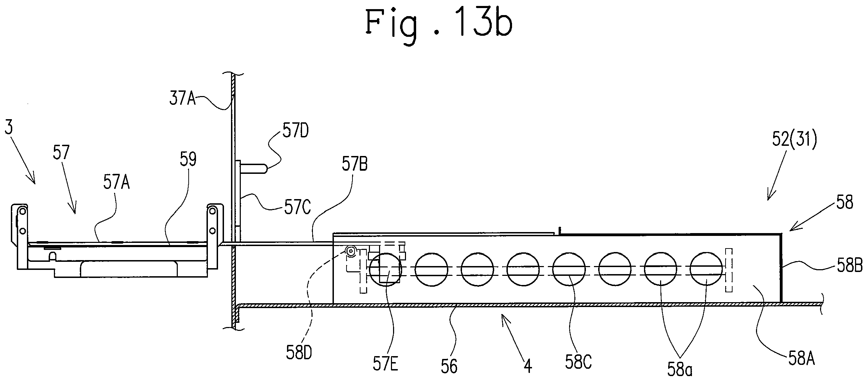

The carrying-in device 31 includes a first carrying-in unit 51 installed in the pass box 4 on the left side shown in Fig. 12a and Fig. 12b, and a second carrying-in unit 52 installed in the pass box 4 on the right side shown in Fig. 13a and Fig. 13b. These two carrying-in units 51 and 52 differ from each other only in that they have widths adjusted to the widths of the two kinds of trays 29 and 30 having different widths, while basically having the same configuration. As shown in Fig. 12a and Fig. 12b, the first carrying-in unit 51 includes a first movable member 53 that can move the first tray 29, which is placed on the first movable member 53 in the pass box 4, toward the isolator 3 side, and a first support member 54 that movably supports the first movable member 53.

The first movable member 53 includes a first mounting member 53A that is a plate like member having a rectangular shape in planer view and allows substantially all the area of the first tray 29 excepting a rear end part to be mounted thereon, and a first interlocking member 53B that is a plate like member having a rectangular shape in planer view extending from one end of the first mounting member 53A, allows the rear end part of the first tray 29 to be mounted thereon, and is interlocked with the first support member 54 side. The first mounting member 53A and the first interlocking member 53B respectively have a large number of holes 53a and a large number of holes 53b, through which air flows pass. The first interlocking member 53B has a width slightly larger than the width of the first mounting member 53A. Provided at a portion of the first interlocking member 53B close to the first mounting member 53A side is a first vertical plate 53C that is provided upstanding to abut a rear end in the carrying-in direction of the first tray 29 to block the movement of the first tray 29 rearward in the carrying-in direction of the first tray 29. A first handle 53D is attached to an upper part of a rear surface in the front-rear direction (carrying-in direction) of the first vertical plate 53C. A plate 55 is arranged at a position just beneath the first movable member 53 when the first movable member 53 has moved into the isolator 3, and has a large number of holes 55A through which air flows pass.

The first support member 54 includes a pair of first side plates 54A, 54A provided upstanding with an interval therebetween in the left-right direction (width direction) on a base plate 56 of the pass box 4, an L-shaped first cover member 54B that is configured to cover an area including a rear half in the front-rear direction of an upper side between the first side plates 54A, 54A and a rear end in the front-rear direction between the first side plates 54A, 54A, a pair of rod-shaped first guide members 54C, 54C that are supported at a certain height from the base plate 56 of the pass box 4 and arranged with a certain interval therebetween in the left-right direction, and a pair of left and right first rotating rollers 54D, 54D that contact a lower surface of the first movable member 53 to support the same during movement of the first movable member 53. First tubular bodies 53E, 53E, through which the pair of first guide members 54C, 54C extend so as to be movable therealong, are connected to a lower surface of a rear end part in the front-rear direction of the first movable member 53. The first side plates 54A, 54A and the first cover member 54B respectively have a large number of holes 54a and a large number of holes 54b, through which air flows pass.

The second carrying-in unit 52 also includes a second movable member 57 that can move the second tray 30, which is placed on the second movable member 57 in the pass box 4, toward the isolator 3 side, and a second support member 58 that movably supports the second movable member 53.

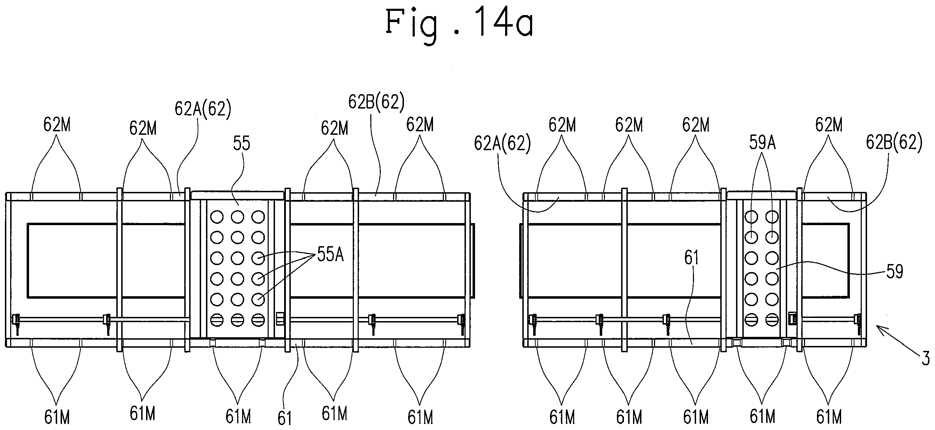

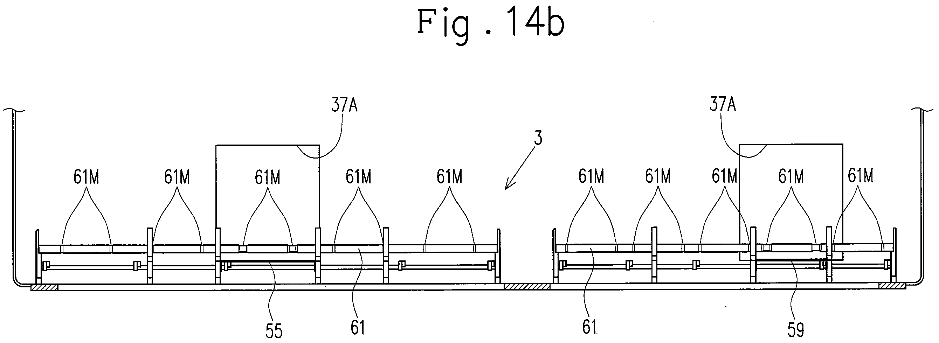

The second movable member 57 includes a second mounting member 57A that is a plate like member having a rectangular shape in planer view and allows substantially all the area of the second tray 30 excepting a rear end part to be mounted thereon, and a second interlocking member 57B that is a plate like member having a rectangular shape in planer view extending from one end of the second mounting member 57A, allows the rear end part of the second tray 30 to be mounted thereon, and is interlocked with the second support member 58 side. The second mounting member 57A and the second interlocking member 57B respectively have a large number of holes 57a and a large number of holes 57b, through which air flows pass. The second interlocking member 57B has a width in the left-right direction slightly larger than the second mounting member 57A. Provided at a portion of the second interlocking member 57B close to the second mounting member 57A side is a second vertical plate 57C that is provided upstanding to abut a rear end in the front-rear direction of the second tray 30 to block the movement of the second tray 30 rearward in the front-rear direction of the second tray 30. A second handle 57D is attached to an upper part of a rear surface in the front-rear direction of the second vertical plate 57C. A plate 59 is arranged at a position just beneath the second movable member 57 when the second movable member 57 has moved into the isolator 3, and has a large number of holes 59A (see Fig. 14) through which air flows pass.

The second support member 58 includes a pair of left and right second side plates 58A, 58A provided upstanding with an interval therebetween in the left-right direction (width direction) on the base plate 56 of the pass box 4, an L-shaped second cover member 58B that is configured to cover an area including a rear half in the front-rear direction of an upper side between the second side plates 58A, 58A and a rear end in the carrying-in direction between the second side plates 58A, 58A, a pair of rod-shaped second guide members 58C, 58C that are supported at a certain height from the base plate 56 of the pass box 4 and arranged with a certain interval therebetween in the left-right direction, and a pair of left and right second rotating rollers 58D, 58D that contact a lower surface of the second movable member 57 to support the same during movement of the second movable member 57. Second tubular bodies 57E, 57E, through which the pair of second guide members 58C, 58C extend so as to be movable therealong, are connected to a lower surface of a rear end part in the front-rear direction of the second movable member 57. The second side plates 58A, 58A and the second cover member 58B respectively have a large number of holes 58a and a large number of holes 58b, through which air flows pass.

For example, when the first movable member 57 is to be moved into the isolator 3, the first handle 53D is grabbed through a given one of the glove ports G1 to G2 and pulled manually toward the isolator 3 side. Fig. 13a and Fig. 13b show the state where the second movable member 57 has been moved to the isolator 3 side. At this moment, as described above, the second tubular bodies 57E, 57E are moved while being guided by the pair of left and right second guide members 58C, 58C, with the lower surface of the second movable member 57 being supported by the pair of left and right second rotating rollers 58D, 58D, so that the second movable member 57 is smoothly moved toward the isolator 3 side while maintaining the horizontal direction thereof. Although the second cover 37 is not shown in Fig. 12a, Fig. 12b, Fig. 13a, and Fig. 13b, the handle 37H of the second cover 37 is grabbed to thereby slide the second cover 37 in the lateral direction to the opening position in an actual operation, so that the first movable member 53 or the second movable member 57 can be moved through the thus opened isolator opening 37A.

As shown in Fig. 11a and Fig. 11b, it is possible to efficiently house the containers in the tray by housing a plural kinds of containers in a tray (the first tray 29 in Figures). As a result, it is possible to reduce the number of times that the containers are carried into the isolator 3, and shorten the time for carrying in the containers corresponding to such reduction. In addition, it is possible to suppress contamination inside of the isolator 3 due to the large amount of air entering the isolator 3 from the pass box 4, because the tray can be instantly carried into the isolator 3 through the small isolator opening 37A which is opened with the second cover 37 moved at the opening position.

The thus configured isolator 3 includes guide units 60, 60 located therein, which are configured to move the first tray 29 or the second tray 30 carried into the isolator 3 by the first movable member 53 or the second movable member 57 while guiding them in a direction (left-right direction) orthogonal to (or crossing) the carrying-in direction (front-rear direction). As shown in Fig. 14a, Fig. 14b, and Fig. 15, each of the guide units 60 is constituted by a pair of front-rear rod shaped members 61 and 62 that extend in the left-right direction with an interval therebetween in the front-rear direction in the isolator 3, and the pair of left and right rotating rollers 29C, 29C and 29D, 29D at the front and rear ends of the first tray 29 or the pair of rotating rollers 30C, 30C and 30D, 30D at the front and rear ends of the second tray 30 in order to engage with the pair of front-rear rod-shaped members 61 and 62.

The rod shaped member 61 on the front side is formed by a single rod-shaped member extending in the left-right direction from a portion corresponding to the isolator opening 37A so that when, for example, the first tray 29 is carried into the isolator 3 by the first carrying-in unit 51, the rod shaped member 61 on the front side can engage with the pair of left and right rotating rollers 29C, 29C at the front end of the first tray 29. A portion corresponding to the isolator opening 37A of the rod shaped member 62 on the rear side is omitted so as to allow passing of the first tray 29, for example, when the first tray 29 is carried into the isolator 3 by the first carrying-in unit 51. The rod shaped member 62 on the rear side is constituted by a left-side rod-shaped member 62A extending from a portion in proximity to the left end of the isolator opening 37A toward the left side, and a right-side rod-shaped member 62B extending from a portion in proximity to the right end of the isolator opening 37A toward the right side. Accordingly, in Fig. 15, when the second tray 30 is carried into the isolator 3 from the pass box 4 on the right side, the pair of left and right rotating rollers 30C, 30C at the front end of the second tray 30 come into engagement with the rod shaped member 61 on the front side from above. In this engaging state, the second robot arm 12 holds the second tray 30 and moves the same toward one side in the left-right direction to thereby allow the pair of left and right rotating rollers 30D, 30D at the rear end of the second tray 30 to come into engagement with the rod shaped member 62A on the rear left side or the rod shaped member 62B on the rear right side. Whereby, the second tray 30 can be smoothly moved to a certain position in the left-right direction while the rotating rollers 30C, 30C and 30D, 30D at the front and rear ends are being rotated along the rod shaped members 61 and 62 on the front and rear sides. When the second tray 30 is to be moved to a far position from the isolator opening 37A, for example, to the left end, the second tray 30 can be moved thereto by transferring of the second tray 30 from the second robot arm 12 at the right end to the second robot arm 11 at the center. Fig. 15 shows the state where the first trays 29 are sequentially carried into the isolator 3 from the pass box 4 on the left side, and five first trays 29 in total (in this embodiment, five trays are provided, but two or more numbers of trays may be provided) are arranged in certain positions in the left-right direction. The second trays 30 from the pass box 4 on the right side can be also arranged with five second trays 30 being aligned in the left-right direction. The rod shaped members 61 and 62 respectively have a pair of grooves 61M and a pair of grooves 62M at each of five places with the same pitch as the rotating rollers. The rotating rollers 29C, 20C or 30C, 30C fit into the front grooves 61M, and the rotating rollers 29D, 29D or 30D, 30D fit into the rear grooves 62M, so that each of the first trays 29 and each of the second trays 30 can be positioned while not being unintentionally moved in the left-right direction. Fig. 15 shows the five first trays 29 respectively fitting into the grooves 61M and 62M. In this arrangement, no rod shaped members are provided on the rear side for the first tray 29 and the second tray 30 positioned at the center. Therefore, the rotating rollers 29D, 29D and 30D, 30D on the rear side cannot fit into the grooves. The pair of grooves 61M, 61M on the front side formed at the center thus have a depth larger than the remaining four pairs of grooves 61M, 61M so as to increase the fitting engagement force to securely prevent unintentional movement of the trays in the left-right direction. In Fig. 15, the containers and the partitioning plates to be housed in the first tray 29 and the second tray 30 are omitted.

Each of the isolator opening 37A and the decontamination chamber opening 35A has such a height as to allow a container having a largest dimension (herein HYPERFlask manufactured by Corning Incorporated) capable of culturing cells to pass therethrough. It is preferable that the isolator opening 37A and the decontamination chamber opening 35A have a width in the left-right direction being slightly larger than the width in the left-right direction of the first tray 29 and the second tray 30, so that the first tray 29 and the second tray 30 can be passed therethrough. The opening 34K of the clean bench chamber R1 also has such a height as to allow a container having a largest dimension (herein HYPERFlask manufactured by Corning Incorporated) capable of culturing cells to pass therethrough. Such height setting enables the size of each opening to be minimized and air flows to be desirably controlled, while enabling all the kinds of handled container to be carried into the respective chambers. As a result, it is possible to more securely avoid occurrence of troubles such as contamination.

The cell treatment apparatus according to the present invention is not limited to the aforementioned embodiment, and various modifications can be made without departing from the gist of the present invention.

The aforementioned embodiment was described by taking, for example, the case where the two robot arms 6 and 7 are provided in the observation section 8, and the three robot arms 10, 11, and 12 are provided in the processing section 13. However, it is also possible to apply, to the present invention, the configuration of providing at least one robot arm in the observation section 8 and providing at least one robot arm in the processing section 13.

In the aforementioned embodiment, the isolator 3 is configured to have a horizontally elongated shape as an example, but may be configured to have a square shape or a circular shape. Further, it may be configured to have a bent shape.

The aforementioned embodiment was described by taking, for example, the case where the first cover 35 is configured to be movable in the vertical direction and the second cover 37 is configured to be movable in the lateral direction. However, the moving direction of each of the first cover 35 and the second cover 37 may be set to any direction.

The description of the aforementioned embodiment was made for the apparatus to produce cultured cell products, which is configured to culture cells and subdivide cultured cells into products. However, the present invention is also applicable to an apparatus to culture cells, which is configured to perform only cell culturing, or applicable to a product manufacturing apparatus, which is configured to subdivide cultured cells into products.

The aforementioned embodiment was described by taking, for example, the case where the two pass boxes 4, 4 are provided. However, the present invention is also applicable to the case where one pass box, or three or more pass boxes are provided.

The aforementioned embodiment was described by taking, for example, the case where each of the trays 29 and 30 has a rectangular shape in planer view. However, the trays 29 and 30 may have any shape, such as a square shape, a polygonal shape, a circular shape or an elliptical shape.

The configuration and action of the aforementioned embodiment will be summarized below. The cell treatment apparatus according to the present embodiment includes: an isolator 3 that has an inner space maintained in aseptic conditions and is configured to treat cells in the inner space; trays 29 and 30 that are each configured to house plural kinds of articles for use in treatment of cells in the inner space of the isolator 3, while positioning them; and pass boxes 4, 4 that are configured to respectively carry the trays 29 and 30 with the plural kinds of containers housed therein into the isolator 3.

According to the above configuration, the plural kinds of containers are housed in each of the trays 29 and 30 while being positioned therein, so that the containers can be efficiently housed in the inner part of each of the trays 29 and 30. As a result, it is possible to reduce the number of times that the containers are carried into the isolator 3, and shorten the time for carrying in the containers corresponding to such reduction.

The isolator 3 may include in the inner space a guide unit 60 that guides the movement of the trays 29 and 30 carried into the inner space of the isolator 3 in a direction crossing the carrying-in direction.

According to such a configuration, the guide unit 60 guides the movements of the trays 29 and 30 carried into the inner space of the isolator 3 in a direction crossing the carrying-in direction, so that a plurality of trays 29 and 30 can be arranged in the inner space of the isolator 3 while being aligned and thereby a large number of cells can be treated.

The isolator 3 may include in the inner space a robot for handling the containers housed in the trays 29 and 30, and the robot is configured to move the trays 29 and 30 carried into the inner space in a direction crossing the carrying-in direction.

According to such a configuration, only carrying-in of the trays 29 and 30 suffices since the robot moves the trays 29 and 30 carried inside in a direction crossing the carrying-in direction.

A plurality of the pass boxes 4, 4 may be provided. With such a configuration including the plurality of pass boxes 4, 4, it is possible to complete carrying-in operations of the trays 29 and 30 in a short time and hence further shorten the time required for carrying in the containers.

### REFERENCE SIGNS LIST

1: Culture vessel
2: Incubator
2A: Casing
2B, 2C: Door
3: Isolator
4: Pass box
5: Mounting table
6, 7: First robot arm
6A: Fixed part
6B: Base part
6C: First arm
6D: Second arm
6E: Third arm
6F: Grip
8: Observation section
9: Product container
10, 11, 12: Second robot arm
13: Processing section
14: Outlet
15: Base member
16: Microscope
17: Centrifuge tube
18: Preparation tank
18A: Cap
19: Conveying apparatus
20: Auxiliary arm
20A: Fixed part
20B: Grip
21: Fixing member
22: Box
23: First disposal part
24: Second disposal part
25: Microscope
26: Centrifuge
27: First box
28: Second box
28A, 28B, 28C: Side wall
29: First tray
29A: Housing body
29B: Partitioning plate
29C, 29D, 30C, 30D: Rotating roller
30: Second tray
31: Carrying-in device
32: Waste liquid tank
33: PBS solution tank
34: Shutter
34K: Opening
35: First cover
35A: Decontamination chamber opening
36: Air cylinder
36A: Piston rod
36B: Cylinder tube
37: Second cover
37A: Isolator opening
37H: Handle
38, 39: Guide rail
38A: Top plate part
38B: Vertical plate part
38C: Horizontal plate part
40: Bracket
41: Moving body
42: Lateral shaft
43: Bearing
44: Spacing member
45: Pin
46: Upper frame body
47: Roller
48: Lower frame body
49, 50: Stopper member
51: First carrying-in unit
52: Second carrying-in unit
53: First movable member
53A: First mounting member
53B: First interlocking member
53C: First vertical plate
53D: First handle
53E: First tubular body
53a, 53b: Hole
54: First support member
54A: First side plate
54B: First cover member
54C: First guide member
54D: First rotating roller
54a, 54b: Hole
55: Plate
56: Base plate
57: Second movable member
57A: Second mounting member
57B: Second interlocking member
57C: Second vertical plate
57D: Second handle
57E: Second tubular body
57a, 57b: Hole
58: Second support member
58A: Second side plate
58B: Second cover member
58C: Second guide member
58D: Second rotating roller
58a, 58b: Hole
59: Plate
60: Guide unit
61, 62: Rod shaped member
62A: Left-side rod-shaped member
62B: Right-side rod-shaped member
G1, G2: Glove port
R2: Decontamination chamber

## Claims

1. A cell treatment apparatus comprising:
an isolator that has an inner space maintained in aseptic conditions and is configured to treat cells in the inner space;
trays that are each configured to house plural kinds of articles for use in treatment of cells in the inner space of the isolator, while positioning them; and
a pass box that is configured to carry the trays with the plural kinds of articles housed therein into the inside of the isolator.

2. The cell treatment apparatus according to claim 1,
wherein the isolator includes in the inner space a guide unit that guides the movements of the trays carried into the inner space of the isolator in a direction crossing the carrying-in direction.

3. The cell treatment apparatus according to claim 2,
wherein the isolator includes in the inner space a robot for handling the containers housed in the trays carried from the pass box, and the robot is configured to move the trays carried into the inner space in a direction crossing the carrying-in direction.

4. The cell treatment apparatus according to any one of claims 1 to 3,
wherein a plurality of the pass boxes are provided.
